# EUROPÄISCHE PATENTSCHRIFT

(11) **EP 2 673 633 B1**
(45) Veröffentlichungstag und Bekanntmachung des Hinweises auf die Patenterteilung: **04.05.2016**
(21) Anmeldenummer: 12709844.0
(22) Anmeldetag: 15.03.2012
(51) Int. Cl.: G01N 33/34

(54) **VERFAHREN UND VORRICHTUNG ZUR ERFASSUNG VON PARAMETERN EINER DURCH- ODER UMLAUFENDEN MATERIALBAHN IN EINER MATERIALVERARBEITUNGSMASCHINE**
METHOD AND DEVICE FOR DETECTING PARAMETERS OF A TRAVERSING OR CIRCULATING MATERIAL WEB IN A MATERIAL PROCESSING MACHINE
PROCÉDÉ ET DISPOSITIF POUR LA DÉTECTION DE PARAMÈTRES D'UNE BANDE DE MATIÈRE QUI TRAVERSE UNE MACHINE DE TRAVAIL DE MATIÈRE OU CIRCULE DANS CETTE MACHINE

(30) Priorität: 30.03.2011 DE 102011006391
(43) Veröffentlichungstag der Anmeldung: 18.12.2013
(73) Patentinhaber: Siemens Aktiengesellschaft, 80333 München (DE)
(72) Erfinder: LANGE, Peter, 94469 Deggendorf (DE); MICHAELIS, Gerd, 91096 Möhrendorf (DE); SCHWARZER, Stefan, 82024 Taufkirchen (DE); ZIROFF, Andreas, 80469 München (DE); MERKEL, Christian, 91052 Erlangen (DE); STUKENKEMPER, Alexander, Cumming 30041-1150 (US)
(86) Internationale Anmeldenummer: PCT/EP2012/054509
(87) Internationale Veröffentlichungsnummer: WO 2012/130618

(56) Entgegenhaltungen:
- EP-A2- 1 985 990
- WO-A1-91/17435
- WO-A1-96/11395
- DE-A1- 19 707 691
- GB-A- 2 082 323
- US-A- 4 496 428
- US-A- 4 501 642
- BRODEUR P H ET AL: "Paper stiffness monitoring using laser ultrasonics", ULTRASONICS SYMPOSIUM, 1996. PROCEEDINGS., 1996 IEEE SAN ANTONIO, TX, USA 3-6 NOV. 1996, IEEE, NEW YORK, NY, USA, Bd. 2, 3. November 1996 (1996-11-03), Seiten 1043-1046, XP010217617, DOI: 10.1109/ULTSYM.1996.584170 ISBN: 978-0-7803-3615-5

## Beschreibung

Die Erfindung betrifft ein Verfahren zur Erfassung von Parametern einer durch- oder umlaufenden Materialbahn in einer Materialverarbeitungsmaschine gemäß dem Oberbegriff des Patentanspruchs 1. Weiterhin betrifft die Erfindung eine Vorrichtung zur Erfassung von Parametern einer durch- oder umlaufenden Materialbahn in einer Materialverarbeitungsmaschine gemäß dem Oberbegriff des Patentanspruchs 3.

Üblicherweise sind in Maschinen zur Herstellung und weiteren Verarbeitung von Materialbahnen, beispielsweise Papierbahnen, Zugmesseinrichtungen angeordnet, welche eine Zugkraft und/oder eine Zugspannung erfassen. Eine Erfassung dieser Werte ist notwendig, um eine konstante Bahnspannung der Materialbahn während des Herstellungs- oder Verarbeitungsprozesses zu ermöglichen, welche für eine hohe Produktqualität und Produktivität unabdingbar ist. Bei herkömmlichen Messeinrichtungen erfolgt die Messung mittels einer Kraftübertragung an einem Zugkraftaufnehmer, welcher vorzugsweise an einem Walzenlager der Maschine angeordnet ist. Ein solcher in mechanischer Wirkverbindung zur Maschine stehender Messaufnehmer unterliegt physikalischen äußeren Einflüssen, wie beispielsweise Temperaturschwankungen, mechanischen Schwingungen der Maschine, einer Verformung von Walzen der Maschine und/oder Unwuchten, welche eine Messgenauigkeit herabsetzen oder Messergebnisse verfälschen.

DE 197 07 691 A1 offenbart ein Verfahren und eine Vorrichtung zur Messung einer Zugspannungsverteilung über eine Breite eines Metallbandes, wobei mittels eines elektromagnetischen Feldes eine Kraft auf das Metallband ausgeübt wird. Die dadurch bewirkte Auslenkung des Metallbandes wird gemessen und zur Berechnung der Zugspannungsverteilung verwendet.

GB 2 082 323 A offenbart ein Verfahren und eine Vorrichtung zur Messung einer Zugspannung in einer Materialbahn, wobei mittels Ultraschall Transversalwellen in der Materialbahn erzeugt werden, die Ausbreitungsgeschwindigkeit dieser Wellen gemessen wird und daraus die Zugspannung ermittelt wird.

EP 1 985 990 A2 offenbart ein Verfahren und eine Vorrichtung zur Bestimmung der Festigkeit einer Faserstoffbahn aus einer für einen Elastizitätsmodul der Faserstoffbahn repräsentativen Größe. Diese Größe wird mittels Ultraschallsignalen ermittelt, mit denen die Faserstoffbahn beaufschlagt wird.

DE 10 2007 032 095 A1 offenbart eine Wickelvorrichtung zum Abrollen einer Materialbahn von einem Volltambour mit einer Sensoreinrichtung zum Erfassen von Schwingungen im Wesentlichen senkrecht zu einer Rollenachse einer Materialbahnrolle.

WO 96/11396 A1 offenbart ein System zur Messung elastischer Eigenschaften einer bewegten Papierbahn. Dabei wird in der Papierbahn eine Ultraschallwelle erzeugt und deren Ausbreitungsgeschwindigkeit in der Papierbahn ermittelt.

US 2001/0003112 A1 offenbart ein Verfahren zur Erfassung von Vibrationen einer Walze, insbesondere an einer Oberfläche der Walze.

US 6 792 807 B2 offenbart ein Verfahren und eine Vorrichtung zur Erkennung von Versiegelungen einer bewegten Kunststofffolie zur Tütenherstellung. Dabei wird eine Kraft auf die Folie ausgeübt, von einem Kraftsensor erfasst und mittels einer Steuereinheit ausgewertet.

US 6 324 912 B1 offenbart ein System zur Erkennung von Fehlstellen in einem Medium mittels eines aus der Relativbewegung des Systems und des Mediums resultierenden akustischen Dopplereffekts. Dabei wird ein akustisches Signal durch das Medium geleitet und ein dopplerverschobendes Signal erfasst und ausgewertet.

US 4 688 423 A offenbart ein System zur Messung der Geschwindigkeit von Vibrationen in einer bewegten Materialbahn, insbesondere einer Papierbahn. Dabei werden in der Materialbahn Vibrationen erzeugt und deren Ausbreitungsgeschwindigkeit ermittelt.

WO 91/17435 A1 offenbart ein Verfahren zur Bestimmung des Elastizitätsmoduls eines bewegten flexiblen Materials. Dabei wird das Material einer Ultraschallwelle ausgesetzt und die Streuung der Ultraschallwelle durch das Material und die Ausbreitungsgeschwindigkeit der Ultraschallwelle werden erfasst und ausgewertet.

US 5 025 665 A offenbart ein System zur berührungslosen Messung einer Materialstärke in einer Materialbahn. Dabei wird mittels eines ersten Laserstrahls in dem Material eine Ultraschallwelle erzeugt und die Ausbreitungsgeschwindigkeit der Ultraschallwelle wird mittels eines zweiten auf das Material gerichteten Laserstrahls ermittelt und ausgewertet.

Der Erfindung liegt daher die Aufgabe zugrunde, ein verbessertes Verfahren und eine verbesserte Vorrichtung zur Erfassung von Parametern einer durch- oder umlaufenden Materialbahn in einer Materialverarbeitungsmaschine anzugeben.

Hinsichtlich des Verfahrens wird die Aufgabe erfindungsgemäß durch die im Anspruch 1 angegebenen Merkmale gelöst. Hinsichtlich der Vorrichtung wird die Aufgabe erfindungsgemäß durch die im Anspruch 3 angegebenen Merkmale gelöst.

Vorteilhafte Weiterbildungen der Erfindung sind Gegenstand der Unteransprüche.

Beim Verfahren zur Erfassung von Parametern einer durch- oder umlaufenden Materialbahn in einer Materialverarbeitungsmaschine werden erfindungsgemäß zumindest ein Schwingungsparameter zumindest einer an der Materialbahn auftretenden Transversalschwingung mittels zumindest eines Sensors berührungslos erfasst und zumindest ein aus diesem Schwingungsparameter resultierender Parameter der Materialbahn ermittelt. Dadurch ist eine verschleißfreie oder nahezu verschleißfreie Erfassung von Parametern der Materialbahn ermöglicht. Insbesondere ist der berührungslos arbeitende Sensor gegenüber äußeren Einflüssen, wie beispielsweise Temperaturschwankungen, mechanischen Schwingungen der Maschine, einer Verformung von Walzen der Maschine und/oder Unwuchten, unempfindlich. Dadurch kann eine Messgenauigkeit gesteigert werden und daraus resultierend kann eine konstante Bahnspannung der Materialbahn während des Herstellungs- oder Verarbeitungsprozesses eingestellt werden, wodurch die Qualität und die Herstellungsgeschwindigkeit der Materialbahn verbessert sind.

In einer bevorzugten Ausführungsform werden als Transversalschwingungen durch Selbsterregung in Längs- und/oder Querrichtung der Materialbahn auftretende Schwingungen erfasst. Diese Selbsterregung kann beispielweise durch Vibrationen der Materialverarbeitungsmaschine selbst oder durch Unwuchten der Walzen, über welche die Materialbahn in der Materialverarbeitungsmaschine geführt wird, erfolgen.

In einer alternativen Ausführungsform werden die in Längs- und/oder Querrichtung auftretenden Transversalschwingungen der Materialbahn fremderregt. Eine solche Fremderregung ist beispielweise anwendbar, wenn mittels einer Selbsterregung keine hinreichend genauen Schwingungsparameter erfassbar sind. Dies kann beispielweise aufgrund einer falschen Frequenz, einer geringen Bandbreite, einer niedrigen Amplitude und/oder einer mangelnden Wiederholbarkeit der Selbsterregung notwendig werden.

In einer vorteilhaften Ausführungsform erfolgt die Fremderregung von Transversalschwingungen der Materialbahn durch Aufbringen eines Schalldrucks. Dabei wird ein Schalldruck mittels eines geeigneten Mittels zumindest abschnittsweise auf die Materialbahn gerichtet oder geleitet, wobei Amplitude, Richtwirkung, Frequenz, Bandbreite, Pulsform und/oder Pulswiederholrate variabel an die jeweilige Materialbahn und die zu erzeugenden Transversalschwingungen anpassbar ist.

In einer vorteilhaften Ausführungsform erfolgt die Fremderregung von Transversalschwingungen der Materialbahn durch mechanische Anregung der Materialbahn. Dabei werden mittels geeigneter Mittel gezielt Vibrationen auf die Materialbahn aufgeprägt, welche Transversalschwingungen bewirken.

Besonders vorteilhafterweise werden als Parameter der Materialbahn eine Zugkraft und/oder -spannung in Bewegungsrichtung der Materialbahn, eine Geschwindigkeit, ein E-Modul in Bewegungsrichtung der Materialbahn, eine relative Feuchtigkeit und/oder eine Dicke der Materialbahn mittels eines vorgegebenen, insbesondere mathematischen Berechnungsmodells der eingespannten und schwingenden Materialbahn ermittelt. Anhand dieser Parameter kann eine konstante Bahnspannung der Materialbahn während des Herstellungs- oder Verarbeitungsprozesses eingestellt werden.

Bei der Vorrichtung zur Erfassung von Parametern einer durch- oder umlaufenden Materialbahn in einer Materialverarbeitungsmaschine sind mittels zumindest eines Sensors mindestens ein Schwingungsparameter zumindest einer an der Materialbahn auftretenden Transversalschwingung berührungslos erfassbar und zumindest ein aus diesem Schwingungsparameter resultierender Parameter der Materialbahn mittels einer Steuereinheit ermittelbar. Dadurch ist eine durch äußere Einflüsse unbeeinflusste Erfassung der Schwingungsparameter der Transversalschwingungen ermöglicht.

Besonders bevorzugt ist eine Mehrzahl von Sensoren längs und/oder quer zur Bewegungsrichtung der Materialbahn angeordnet und auf diese ausgerichtet. Mittels einer Mehrzahl von Sensoren, welche auf unterschiedliche Oberflächenbereiche der Materialbahn ausgerichtet sind, kann auf einfache Weise eine Ausbreitungsrichtung und eine Ausbreitungsgeschwindigkeit der Transversalschwingungen erfasst werden.

Vorteilhafterweise sind der Sensor oder die Sensoren als Radarsensor, Dopplerradarsensor, Ultraschallsensor und/oder Lasersensor ausgebildet. Diese berührungslos wirkenden Sensoren sind gegenüber mechanischen Einflüssen, beispielsweise Vibrationen, Staubablagerungen, Temperaturschwankungen und/oder hoher Luftfeuchtigkeit unempfindlich und arbeiten verschleißfrei.

Ausführungsbeispiele der Erfindung werden anhand von Zeichnungen näher erläutert.

Dabei zeigen:
- FIG 1: schematisch eine Materialverarbeitungsmaschine gemäß dem Stand der Technik,
- FIG 2: schematisch eine Materialverarbeitungsmaschine mit einer erfindungsgemäßen Vorrichtung zur Erfassung von Parametern einer durch- oder umlaufenden Materialbahn und
- FIG 3: schematisch ein Verfahrensablaufdiagramm eines erfindungsgemäßen Verfahrens.

Einander entsprechende Teile sind in allen Figuren mit den gleichen Bezugszeichen versehen.

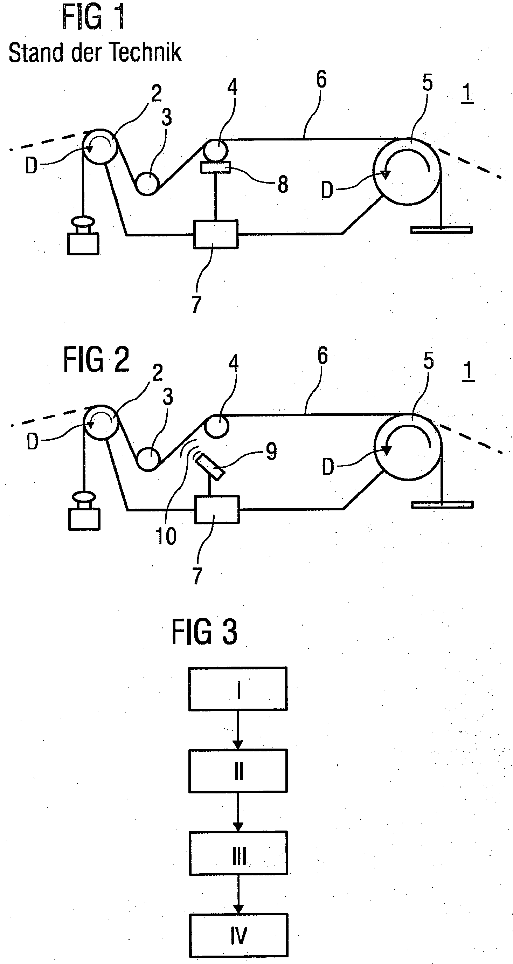

In Figur 1 ist schematisch eine Materialverarbeitungsmaschine 1 gemäß dem Stand der Technik dargestellt. Eine solche Materialverarbeitungsmaschine 1 umfasst eine Mehrzahl von Walzen 2, 3, 4, 5, welche jeweils einen unterschiedlichen Durchmesser aufweisen können, über die eine Materialbahn 6 geführt wird. Dabei können einzelne Walzen 2, 5 aktiv antreibbar sein, wobei an den einzelnen angetriebenen Walzen 2, 5 unterschiedliche Rotationsgeschwindigkeiten oder Drehzahlen D einstellbar sind. Durch Variation der Rotationsgeschwindigkeiten oder Drehzahlen D kann eine Bahnspannung der Materialbahn 6 beeinflusst werden. Eine Regelung der Rotationsgeschwindigkeiten oder Drehzahlen D kann manuell durch das Bedienpersonal oder automatisch mittels einer Steuereinheit 7 erfolgen.

Herkömmlicherweise wird eine Zugkraft und/oder -spannung in Bewegungsrichtung (auch Laufrichtung oder Längsrichtung genannt) der Materialbahn 6 mittels eines Zugkraftaufnehmers 8, welcher vorzugsweise an einem Walzenlager der Walze 4 der Materialverarbeitungsmaschine 1 angeordnet ist, ermittelt. Ein solcher, in mechanischer Wirkverbindung zur Materialverarbeitungsmaschine 1 stehender Zugkraftaufnehmer 8 unterliegt physikalischen äußeren Einflüssen, wie beispielsweise Temperaturschwankungen, mechanischen Schwingungen der Materialverarbeitungsmaschine 1, einer Verformung von Walzen der Materialverarbeitungsmaschine 1, Verschmutzung und/oder Unwuchten, welche eine Messgenauigkeit herabsetzen oder Messergebnisse verfälschen.

Anhand der ermittelten Zugkraft Z und/oder -spannung kann die Drehzahl D der angetriebenen Walzen 2, 5 manuell oder automatisch eingestellt werden, so dass eine konstante Bahnspannung der Materialbahn 6 während des Herstellungs- oder Verarbeitungsprozesses ermöglicht ist.

Die Materialbahn 6 ist vorzugsweise als herkömmliche Papierbahn ausgebildet und die Materialverarbeitungsmaschine 1 ist beispielsweise als Papiermaschine, Streichmaschine, Umroller und/oder Rollenschneidmaschine ausbildet.

In Figur 2 ist schematisch eine Materialverarbeitungsmaschine 1 mit einer erfindungsgemäßen Vorrichtung zur Erfassung von Parametern einer durchlaufenden Materialbahn 6 dargestellt. Alternativ kann die Materialverarbeitungsmaschine für eine umlaufende Materialbahn ausgebildet sein (nicht näher dargestellt). Die erfindungsgemäße Vorrichtung zur Erfassung der Parameter an einer umlaufenden Materialbahn unterscheidet sich nicht wesentlich von der zur Erfassung von Parametern an einer durchlaufenden Materialbahn 6. Lediglich die Position der Vorrichtung kann verschieden sein. Die Komponenten sind im Wesentlichen identisch.

Die Materialverarbeitungsmaschine 1 gemäß Figur 2 entspricht im Wesentlichen der in Figur 1 dargestellten Materialverarbeitungsmaschine 1, mit dem Unterschied, dass kein Zugkraftaufnehmer 8 am Walzenlager der Walze 4 angeordnet ist.

Eine Mehrzahl von Sensoren 9 ist längs und/oder quer zur Bewegungsrichtung der Materialbahn 6 angeordnet, wobei ein Erfassungsbereich 10 der Sensoren 9 auf die Materialbahn 6 ausgerichtet ist und dort auftretende, so genannte Transversalschwingungen erfasst. Besonders vorteilhafterweise kann mittels mehrerer Sensoren 9, welche jeweils auf unterschiedliche Oberflächenbereiche der Materialbahn 6 ausgerichtet sind, auf einfache Weise eine Ausbreitungsrichtung und/oder eine Ausbreitungsgeschwindigkeit der Transversalschwingungen erfasst werden. Die Ausbreitungsgeschwindigkeit der Transversalschwingungen steht dabei in direkter Beziehung zur Bahnspannung der Materialbahn 6, so dass diese Bahnspannung aus der Ausbreitungsgeschwindigkeit der Transversalschwingungen ermittelt werden kann. Die Sensoren 9 können unterschiedliche Schwingungsparameter der Transversalschwingungen, wie beispielweise eine Frequenz, eine Amplitude und/oder eine Phase, erfassen. Dazu sind die Sensoren 9 je nach Richtung der Wellenausbreitung der Transversalschwingungen längs oder quer zur Bewegungsrichtung der Materialbahn 6 ausgerichtet.

Die Materialbahn 6 ist in Zugrichtung an ihren beiden Stirnseiten zwischen den Walzen 2 und 5 eingespannt. An den Längsseiten ist die Materialbahn 6 nicht eingespannt. Auf der mit einer vorgebbaren Geschwindigkeit durchlaufenden Materialbahn 6 können sich herkömmliche Transversalschwingungen in Lauf- oder Bewegungsrichtung (= Längsrichtung), somit zwischen den eingespannten Stirnseiten, und quer zur Lauf- oder Bewegungsrichtung (= Querrichtung), somit zwischen den Längsseiten ausbreiten. In Längsrichtung können aufgrund der Einspannung und der Bahnbewegung laufende und stehende Wellen von Transversalschwingungen auftreten. In Querrichtung können laufende Wellen der Transversalschwingungen auftreten, die an den Längsseiten reflektiert werden können.

Die Transversalschwingungen der Materialbahn 6 können selbst- oder fremderregt entstehen. Selbsterregte Transversalschwingungen entstehen beispielsweise aufgrund von Vibrationen der Materialverarbeitungsmaschine 1 oder einzelner Maschinenteile, beispielsweise durch Unwucht zumindest einer der Walzen 2 bis 5, über welche die Materialbahn 6 in der Materialverarbeitungsmaschine 1 geführt wird oder durch turbulente Luftströmung an der Materialbahn 6.

Sind mittels einer solchen Selbsterregung keine hinreichend genauen Schwingungsparameter der Transversalschwingungen erfassbar, ist eine Fremderregung anwendbar. Dies kann beispielweise aufgrund einer falschen Frequenz, einer geringen Bandbreite, einer niedrigen Amplitude und/oder einer mangelnden Wiederholbarkeit der Selbsterregung notwendig werden. Dazu werden die fremderregten Transversalschwingungen beispielsweise mittels Schalldrucks oder mechanisch induziert. Dabei wird ein Schalldruck mittels eines geeigneten Mittels zumindest abschnittsweise auf die Materialbahn 6 gerichtet oder geleitet, wobei Amplitude, Richtwirkung, Frequenz, Bandbreite, Pulsform und/oder Pulswiederholrate variabel an die jeweilige Materialbahn 6 und die zu erzeugenden Transversalschwingungen anpassbar ist.

In einer alternativen Ausführungsform erfolgt die Fremderregung von Transversalschwingungen der Materialbahn 6 durch mechanische Anregung der Materialbahn 6. Dabei werden mittels geeigneter Mittel gezielt Vibrationen auf die Materialbahn 6 aufgeprägt, welche Transversalschwingungen bewirken.

Mittels einer Wahl des Einleitungsortes und/oder der Einleitungsrichtung können unterschiedliche Transversalschwingungen gezielt und wiederholbar angeregt werden.

Vorteilhafterweise sind die Sensoren 9 als herkömmliche Radarsensoren, Dopplerradarsensoren, Ultraschallsensoren und/oder Lasersensoren ausgebildet. Diese berührungslos wirkenden Sensoren 9 sind gegenüber mechanischen Einflüssen, beispielweise Vibrationen, Staubablagerungen, Temperaturschwankungen und/oder hoher Luftfeuchtigkeit unempfindlich und arbeiten verschleißfrei.

Besonders vorteilhafterweise sind als Sensoren 9 Dopplerradarsensoren einsetzbar, da sie die Amplitude und Phase einer Transversalschwingung eines reflektierenden Materials direkt messen können. Dabei arbeiten diese Dopplerradarsensoren vorzugsweise in einem Frequenzbereich von 77 GHz.

Basierend auf einem vorgegebenen, insbesondere mathematischen Berechnungsmodell der eingespannten und schwingenden Materialbahn 6, welches vorzugsweise in der Steuereinheit 7 hinterlegt ist, können aus den aufgenommenen Schwingungsparametern der Transversalschwingungen als Parameter der Materialbahn 6 eine Zugkraft und/oder -spannung in Bewegungsrichtung der Materialbahn 6, eine Geschwindigkeit, ein E-Modul in Bewegungsrichtung der Materialbahn 6, eine relative Feuchtigkeit und/oder eine Dicke der Materialbahn 6 ermittelt werden.

Für einige dieser Parameter kann es notwendig sein, Sensoren 9 auf mehreren Abschnitten der Materialbahn 6, also zwischen unterschiedlichen Walzenpaaren, anzuordnen und zu verteilen und das mathematische Berechnungsmodell entsprechend zu erweitern, beispielsweise zur Erhaltung des Massenstroms. Dabei sind die Parameter und/oder die Parameterkombinationen, welche mit hinreichender Genauigkeit ermittelbar sind, von der Zahl und der Position der Sensoren 9 sowie von der Ordnung des mathematischen Berechnungsmodells der Materialbahn 6 abhängig.

In Figur 3 ist schematisch ein Verfahrensablaufdiagramm eines erfindungsgemäßen Verfahrens dargestellt. Im Betrieb des Verfahrens wird in einem ersten Verfahrensschritt I zumindest eine Transversalschwingung fremderregt auf die Materialbahn 6 aufgeprägt.

In einem zweiten Verfahrensschritt II wird zumindest ein Schwingungsparameter einer an der Materialbahn 6 auftretenden Transversalschwingung mittels mindestens eines Sensors 9 berührungslos erfasst und an die mit dem Sensor 9 gekoppelte Steuereinheit 7 übermittelt.

In einem dritten Verfahrensschritt III werden anhand des in der Steuereinheit 7 hinterlegten mathematischen Berechnungsmodells der eingespannten, durchlaufenden und schwingenden Materialbahn 6 und der erfassten Schwingungsparameter Parameter der Materialbahn 6 ermittelt.

In einem vierten Verfahrensschritt IV werden die ermittelten Parameter der Materialbahn 6 einem Bedienpersonal der Materialverarbeitungsmaschine 1 visuell angezeigt, wobei das Bedienpersonal die Materialverarbeitungsmaschine 1 manuell steuert oder regelt.

In einer alternativen, bevorzugten Ausführungsform werden die berechneten Parameter der Materialbahn 6 zur automatischen Steuerung und/oder Regelung der Materialverarbeitungsmaschine 1 genutzt.

## Patentansprüche

1. Verfahren zur Erfassung von Parametern einer durch- oder umlaufenden Materialbahn (6) in einer Materialverarbeitungsmaschine (1),
**dadurch gekennzeichnet, dass** zumindest ein Schwingungsparameter zumindest einer an der Materialbahn (6) auftretenden Transversalschwingung mittels zumindest eines Sensors (9) berührungslos erfasst wird und mindestens ein aus diesem Schwingungsparameter resultierender Parameter der Materialbahn (6) ermittelt wird, wobei als zumindest ein Schwingungsparameter einer Transversalschwingung eine Frequenz und/oder eine Amplitude und/oder eine Phase der Transversalschwingung erfasst wird und als mindestens ein Parameter der Materialbahn (6) eine Zugkraft und/oder -spannung in Bewegungsrichtung der Materialbahn (6) und/oder eine Geschwindigkeit und/oder ein E-Modul in Bewegungsrichtung der Materialbahn (6) und/oder eine relative Feuchtigkeit und/oder eine Dicke der Materialbahn (6) ermittelt wird, und wobei als Transversalschwingungen durch Selbsterregung in Längs- und/oder Querrichtung an der Materialbahn (6) auftretende Schwingungen erfasst werden oder die in Längs- und/oder Querrichtung auftretenden Transversalschwingungen der Materialbahn (6) durch mechanische Anregung der Materialbahn (6) fremderregt werden.

2. Verfahren nach Anspruch 1,
**dadurch gekennzeichnet, dass** mindestens ein Parameter der Materialbahn (6) mittels eines in einer Steuereinheit (7) hinterlegten Berechnungsmodells der eingespannten und schwingenden Materialbahn (6) aus zumindest einem erfassten Schwingungsparameter ermittelt wird.

3. Vorrichtung zur Erfassung von Parametern einer durch- oder umlaufenden Materialbahn (6) in einer Materialverarbeitungsmaschine (1),
**gekennzeichnet durch** mindestens einen als Radarsensor, Dopplerradarsensor, Ultraschallsensor oder Lasersensor ausgebildeten Sensor (9) zur berührungslosen Erfassung einer Frequenz und/oder Amplitude und/oder Phase einer an der Materialbahn (6) **durch** Selbsterregung auftretenden oder **durch** mechanische Anregung der Materialbahn (6) fremderregten Transversalschwingung und **durch** eine mit dem mindestens einen Sensor (9) gekoppelte Steuereinheit (7) zur Ermittlung einer Zugkraft und/oder -spannung in Bewegungsrichtung der Materialbahn (6) und/oder einer Geschwindigkeit und/oder eines E-Moduls in Bewegungsrichtung der Materialbahn (6) und/oder einer relativen Feuchtigkeit und/oder einer Dicke der Materialbahn (6) anhand der von dem mindestens einen Sensor (9) erfassten Daten.

4. Vorrichtung nach Anspruch 3,
**dadurch gekennzeichnet, dass** eine Mehrzahl von Sensoren (9) längs und/oder quer zur Bewegungsrichtung der Materialbahn (6) angeordnet und auf diese ausgerichtet sind.

5. Vorrichtung nach einem der Ansprüche 3 oder 4,
**dadurch gekennzeichnet, dass** in der Steuereinheit (7) ein Berechnungsmodell der eingespannten und schwingenden Materialbahn (6) zur Ermittelung einer Zugkraft und/oder -spannung in Bewegungsrichtung der Materialbahn (6) und/oder einer Geschwindigkeit und/oder eines E-Moduls in Bewegungsrichtung der Materialbahn (6) und/oder einer relativen Feuchtigkeit und/oder einer Dicke der Materialbahn (6) anhand der von dem mindestens einen Sensor (9) erfassten Daten hinterlegt ist.

6. Vorrichtung nach einem der Ansprüche 3 bis 5,
**dadurch gekennzeichnet, dass** die Sensoren (9) zwischen zwei benachbart in der Materialverarbeitungsmaschine (1) angeordneten Walzen (2, 3) angeordnet sind oder zwischen mehreren Walzen (2, 3, 4, 5) angeordnet sind.

7. Verwendung des Verfahrens nach einem der Ansprüche 1 oder 2 zur Erfassung von Parametern einer als Papierbahn ausgebildeten Materialbahn (6) in einer als Papiermaschine, Streichmaschine, Umroller und/oder Rollenschneidmaschine ausgebildeten Materialverarbeitungsmaschine (1).

## Claims

1. Method for detecting parameters of a traversing or circulating material web (6) in a material processing machine (1),
**characterised in that** at least one oscillation parameter of at least one transverse oscillation occurring at the material web (6) is contactlessly detected by means of at least one sensor (9), and at least one parameter relating to the material web (6) and resulting from said oscillation parameter is determined, wherein a frequency and/or an amplitude and/or a phase of the transverse oscillation is detected as at least one oscillation parameter of a transverse oscillation, and a tensile force and/or tensile stress in a direction of movement of the material web (6) and/or a speed and/or an elasticity modulus in a direction of movement of the material web (6) and/or a relative humidity and/or a thickness of the material web (6) is determined as at least one parameter of the material web (6), and wherein oscillations occurring in a longitudinal and/or cross direction at the material web (6) due to self-excitation are detected as transverse oscillations, or the transverse oscillations of the material web (6) occurring in a longitudinal and/or cross direction are separately excited by mechanical excitation of the material web (6).

2. Method according to claim 1,
**characterised in that** at least one parameter of the material web (6) is determined from at least one detected oscillation parameter by means of a calculation model of the restrained and oscillating material web (6), said model being stored in a control unit (7).

3. Device for detecting parameters of a traversing or circulating material web (6) in a material processing machine (1),
**characterised by** at least one sensor (9) taking the form of a radar sensor, Doppler radar sensor, ultrasound sensor or laser sensor for contactlessly detecting a frequency and/or amplitude and/or phase of a transverse oscillation which occurs at the material web (6) as a result of self-excitation or is separately excited as a result of mechanical excitation of the material web (6), and by a control unit (7), this being coupled to the at least one sensor (9), for determining a tensile force and/or tensile stress in a direction of movement of the material web (6) and/or a speed and/or an elasticity modulus in a direction of movement of the material web (6) and/or a relative humidity and/or a thickness of the material web (6) on the basis of the data detected by the at least one sensor (9).

4. Device according to claim 3,
**characterised in that** a plurality of sensors (9) are disposed along the material web (6), longitudinally and/or crosswise relative to the direction of movement, and aligned therewith.

5. Device according to one of the claims 3 or 4,
**characterised in that** a calculation model of the restrained and oscillating material web (6) is stored in the control unit (7) for the purpose of determining a tensile force and/or tensile stress in a direction of movement of the material web (6) and/or a speed and/or an elasticity modulus in a direction of movement of the material web (6) and/or a relative humidity and/or a thickness of the material web (6) on the basis of the data detected by the at least one sensor (9).

6. Device according to one of the claims 3 to 5,
**characterised in that** the sensors (9) are arranged between two adjacently disposed rollers (2, 3) in the material processing machine (1) or between a plurality of rollers (2, 3, 4, 5).

7. Use of the method according to one of the claims 1 or 2 for detecting parameters of a material web (6) taking the form of a paper web in a material processing machine (1) taking the form of a paper machine, coating machine, rewinder and/or roll cutting machine.

## Revendications

1. Procédé de détection de paramètres d'une bande ( 6 ) de matière qui passe ou tourne dans une machine ( 1 ) de traitement de matière,
**caractérisé en ce que** l'on détecte sans contact à l'aide d'au moins un capteur ( 9 ) un paramètre d'au moins une oscillation transversale se produisant sur la bande ( 6 ) de matière et on détermine au moins un paramètre de la bande ( 6 ) de matière résultant de ce paramètre d'oscillation, dans lequel on détecte, comme au moins un paramètre d'une oscillation transversale, une fréquence et/ou une amplitude et/ou une phase de l'oscillation transversale et on détermine, comme au moins un paramètre de la bande ( 6 ) de matière, une force de traction et/ou une contrainte de traction dans le sens de déplacement de la bande ( 6 ) de matière et/ou une vitesse et/ou un module E dans le sens de déplacement de la bande ( 6 ) de matière et/ou une humidité relative et/ou une épaisseur de la bande ( 6 ) de matière et dans lequel on détecte, comme oscillations transversales, des oscillations se produisant par auto-excitation dans la direction longitudinale et/ou transversale sur la bande ( 6 ) de matière ou on excite par excitation mécanique indépendante de la bande ( 6 ) de matière les oscillations transversales de la bande ( 6 ) de matière se produisant dans la direction longitudinale et/ou transversale.

2. Procédé suivant la revendication 1,
**caractérisé en ce que** l'on détermine au moins un paramètre de la bande ( 6 ) de matière à l'aide d'un modèle de calcul mémorisé dans une unité ( 7 ) de commande de la bande ( 6 ) de matière serrée et oscillant à partir d'au moins un paramètre d'oscillation détecté.

3. Dispositif de détection de paramètres d'une bande ( 6 ) de matière tournant ou passant dans une machine ( 1 ) de traitement de matière,
**caractérisé par** au moins un capteur ( 6 ) sous la forme d'un capteur radar, d'un capteur radar Doppler, d'un capteur d'ultrasons ou d'un capteur laser, pour la détection sans contact d'une fréquence et/ou d'une amplitude et/ou d'une phase sur la bande ( 6 ) de matière par une oscillation transversale se produisant par auto-excitation ou par excitation mécanique indépendante de la bande ( 6 ) de matière et par une unité ( 7 ) de commande couplée au au moins un capteur ( 9 ) pour la détermination d'une force de traction et/ou d'une contrainte de traction dans la direction de déplacement de la bande ( 6 ) et/ou d'une vitesse et/ou d'un module E dans la direction de déplacement de la bande ( 6 ) et/ou d'une humidité relative et/ou d'une épaisseur de la bande ( 6 ) de matière à l'aide des données détectées par le au moins un capteur ( 9 ).

4. Dispositif suivant la revendication 3,
**caractérisé en ce qu'**une pluralité de capteurs ( 9 ) sont disposés le long de la direction de déplacement de la bande ( 6 ) de matière et/ou transversalement à la direction de déplacement de la bande ( 6 ) de matière et sont dirigés sur celle-ci.

5. Dispositif suivant l'une des revendications 3 ou 4,
**caractérisé en ce qu'**il est mémorisé, au moyen des données détectées par le au moins un capteur ( 9 ), dans l'unité ( 7 ) de commande un modèle de calcul de la bande ( 6 ) de matière serrée et oscillante, pour la détermination d'une force de traction et/ou d'une contrainte de traction dans la direction de déplacement de la bande ( 6 ) de matière et/ou d'une vitesse et/ou d'un module E dans la direction de déplacement de la bande ( 6 ) de matière et/ou d'une humidité relative et/ou d'une épaisseur de la bande ( 6 ) de matière.

6. Dispositif suivant l'une des revendications 3 à 5,
**caractérisé en ce que** les capteurs ( 9 ) sont disposés entre deux rouleaux ( 2, 3 ) voisins dans la machine ( 1 ) de traitement de matière ou entre plusieurs rouleaux ( 2, 3, 4, 5 ) .

7. Utilisation du procédé suivant l'une des revendications 1 ou 2 pour la détection de paramètres d'une bande ( 6 ) de matière constituée sous la forme d'une bande de papier dans une machine ( 1 ) de traitement de matière, sous la forme d'une machine à papier, d'une coucheuse, d'une machine à enrouler et/ou d'une enrouleuse/refendeuse.
